# DEMANDE DE BREVET EUROPEEN

(11) **EP 1 464 680 A2**
(43) Date de publication de la demande: **06.10.2004**
(21) Numéro de dépôt: 03292288.2
(22) Date de dépôt: 17.09.2003
(51) Int. Cl.: C09C 1/00

(54) **Pigment interférentiel et composition cosmétique comportant un tel pigment**

(30) Priorité: 18.09.2002 FR 0211579
(71) Demandeur: L'OREAL, 75008 Paris (FR)
(72) Inventeur: Simon, Jean-Christophe, 162-0842 Tokyo (JP)
(74) Mandataire: Tanty, François

(57) **Abrégé**

La présente invention concerne un pigment interférentiel ayant une structure multicouche, caractérisé par le fait qu'il présente une réflectance spectrale s'écartant d'au plus 20 % de la réflectance spectrale d'un type de matière kératinique pour au moins une partie du spectre visible de 200 nm d'étendue, ce pigment étant caractérisé par le fait que la structure multicouche comporte au moins une couche (3b ; 3c) enrobant en totalité une couche sous-jacente (3a ; 3b).

## Description

La présente invention concerne les compositions cosmétiques ou de soin destinées à être appliquées sur la peau, y compris les muqueuses, et les phanères.

On connaît par la demande internationale WO 00/75240 des pigments interférentiels ayant une structure multicouche, présentant une réflectance spectrale proche d'une réflectance spectrale de référence correspondant à un type de peau prédéterminé, pour au moins une partie du spectre visible.

Ces pigments peuvent être obtenus en déposant sur un polymère soluble dans un solvant donné, présent sur un support flexible, les couches successives de la structure multicouche, par une technique de dépôt sous vide. Ensuite, le film multicouche est fragmenté pour former des plaquettes de petites dimensions constituant les particules du pigment.

De tels pigments permettent de réaliser des compositions imitant la couleur de la peau, afin d'en couvrir et masquer des défauts, par exemple.

Néanmoins, les plaquettes présentent une forme qui favorise la réflexion spéculaire de la lumière et leur brillance doit être atténuée en introduisant dans la composition une charge matifiante.

L'invention vise notamment à perfectionner encore les pigments décrits dans WO 00/75240 et à réaliser des compostions cosmétiques ou de soin permettant par exemple d'imiter au mieux l'aspect de la peau ou des phanères, afin par exemple d'en corriger des défauts, notamment des inhomogénéités de coloration, ou permettant d'atténuer une dominante chromatique indésirable.

L'invention a pour objet, selon l'un de ses aspects, un pigment interférentiel ayant une structure multicouche, ce pigment présentant une réflectance spectrale s'écartant d'au plus 20 % de la réflectance spectrale d'un type de matière kératinique pour au moins une partie du spectre visible de 200 nm d'étendue, ce pigment pouvant se caractériser par le fait que la structure multicouche comporte au moins une couche enrobant en totalité une couche sous-jacente.

Un tel pigment se différencie des pigments obtenus selon la méthode rappelée plus haut et décrite dans WO 00/75240, pour lesquels les différentes couches n'enrobent pas les couches sous-jacentes au niveau des zones de cassure du film multicouche ayant conduit à la formation des plaquettes.

L'invention permet notamment de réaliser plus facilement des particules de pigment avec une forme plus « arrondie », ce qui peut diminuer leur aptitude à réfléchir la lumière de manière spéculaire et leur conférer une meilleure matité, donc leur permettre par exemple de recréer plus fidèlement l'aspect de la peau.

Dans un exemple de mise en oeuvre de l'invention, les particules de pigment présentent chacune une forme générale globulaire, notamment sphérique. Les particules de pigment peuvent comporter un substrat pouvant lui aussi avoir une forme globulaire, notamment sphérique, sur lequel sont déposées les différentes couches de la structure multicouche. Le substrat peut notamment comporter une microsphère d'une matière organique ou minérale, par exemple un verre ou un métal, notamment de l'aluminium.

Le substrat peut être creux ou plein, et présente avantageusement une forme symétrique par rapport à un centre de symétrie, par exemple une forme sphérique ou polyédrique.

La matière kératinique peut être par exemple une peau, notamment une peau caucasienne, métisse, asiatique ou noire.

Dans un exemple de mise en oeuvre de l'invention, la réflectance spectrale du pigment est proche de la réflectance spectrale du type de matière kératinique considéré sur la totalité du spectre visible, c'est-à-dire sur la gamme de longueur d'ondes s'étendant de 400 nm à 700 nm, de 300 nm d'étendue.

La réflectance spectrale du pigment multicouche peut s'écarter, pour une plage du spectre considérée, par exemple pour au moins une plage de 200 nm d'étendue, par exemple pour au moins la plage allant de 500 nm à 700 nm, d'au plus 10 % de la réflectance spectrale du type de matière kératinique, voire d'au plus 5 %, ou d'au plus 2 %.

De préférence, le pigment multicouche est configuré de manière à présenter une valeur de clarté L*, mesurée dans l'espace CIE 1976, sensiblement constante pour des incidences comprises entre -45° et 45°.

La dimension des particules du pigment peut être comprise entre 10 et 150 µm, mieux entre 10 et 50 µm. Par « dimension », on désigne la dimension donnée par la distribution granulométrique statistique à la moitié de la population, dite D50.

Dans un souci d'esthétique, il est préférable que les particules du pigment ne soient pas perceptibles du tout à l'oeil nu à la surface de la composition appliquée sur son support. Il est également souhaitable que les particules de pigment ne soient pas de dimension telle qu'elles créent une sensation d'inconfort sur la peau. L'utilisation de particules de taille inférieure ou égale à 250 µm, et mieux inférieure ou égale à 150 µm, est ainsi privilégiée. La taille des particules pourra dépendre de la nature du support sur lequel la composition est destinée à être appliquée ; certaines parties du corps et du visage pourront par exemple plus facilement que d'autres tolérer une dimension plus grande sans générer d'inconfort.

Le pigment peut comporter au moins une couche réalisée dans un matériau choisi parmi MgF₂, CeF₃, ZnS, ZnSe, Si, Ge, Te, SiO₂, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Fe₂O₃, Pt, Va, Ti, Ta, Zn, la cryolithe, les alliages, les polymères et leurs associations.

L'invention a encore pour objet, selon un autre de ses aspects, une composition comportant, dans un milieu physiologiquement acceptable, au moins un pigment interférentiel ayant une structure multicouche, la réflectance spectrale de la composition s'écartant d'au plus 20 % de la réflectance spectrale d'un type de matière kératinique pour au moins une partie du spectre visible de 200 nm d'étendue, cette composition étant caractérisée par le fait que la structure multicouche comporte au moins une couche enrobant en totalité une couche sous-jacente.

Une telle composition peut comporter un pigment interférentiel ayant une structure multicouche telle que définie précédemment.

Une telle composition peut encore comporter une pluralité de pigments ayant des structures différentes, les pigments pouvant être ou non tous de type interférentiel à structure multicouche.

La composition peut notamment comporter un mélange de pigments, les proportions des différents pigments étant choisies de manière à obtenir la réflectance spectrale souhaitée pour la composition.

La composition peut notamment comporter un mélange de pigments qui permette d'obtenir une réflectance spectrale proche de la réflectance spectrale de référence même en cas de variation de l'indice de réfraction d'une phase de la composition, afin de tenir compte par exemple de l'absorption de sébum par la composition ou de l'évaporation d'un ingrédient. En d'autres termes, le pigment interférentiel maintient une réflectance spectrale qui est proche de la réflectance spectrale de référence, même en cas de variation de l'indice de réfraction.

La structure multicouche est avantageusement configurée de manière à ce que la composition soit sensiblement non goniochromatique pour des incidences comprises entre -45° et 45°, ce qui peut permettre de recréer plus fidèlement l'aspect de la peau.

Par « composition non goniochromatique », on désigne au sens de la présente invention une composition permettant d'obtenir, lorsqu'elle est étalée sur un support, un trajet de couleur dans le plan a*, b* de l'espace colorimétrique CIE 1976 qui correspond à une variation Dh de l'angle de teinte h d'au plus 20°, lorsque l'on fait varier l'angle d'observation par rapport à la normale entre 0° et 80°, pour un angle d'incidence de la lumière de 45°. Le trajet de couleur peut être mesuré par exemple au moyen d'un spectrogonioréflectomètre de marque Instrument Systems et de référence GON 360 GONIOMETER, après que la composition ait été étalée à l'état fluide avec une épaisseur de 300 µm au moyen d'un étaleur automatique sur une carte de contraste de marque ERICHSEN et de référence TYP 24/5, la mesure étant effectuée sur le fond noir de la carte. Le paramètre de clarté L* peut être également sensiblement constant pour des incidences allant de 0° à 50°.

De préférence, la réflectance spectrale de la composition appliquée sur son support s'écarte, pour une plage du spectre considérée, par exemple pour une plage d'au moins 200 nm d'étendue, par exemple pour au moins la plage allant de 500 nm à 700 nm, d'au plus 10 % de la réflectance spectrale de référence et, mieux, d'au plus 5 %, voire encore mieux d'au plus 2 % lorsque l'on cherche à imiter au mieux la couleur de la peau.

L'invention a encore pour objet l'utilisation d'une composition telle que définie plus haut pour dissimuler des imperfections de la peau, notamment des rides, boutons, taches, couperose, veines et points noirs.

L'invention a encore pour objet l'utilisation d'une composition telle que définie plus haut pour atténuer une dominante chromatique de la peau, par exemple le jaune.

L'invention a encore pour objet un procédé de fabrication d'un pigment interférentiel ayant une structure multicouche, caractérisé par le fait qu'il comporte les étapes suivantes :
- définir une réflectance spectrale de référence ayant au moins une portion sensiblement confondue avec la réflectance spectrale d'un type de matière kératinique,
- réaliser la structure multicouche de telle sorte qu'elle comporte au moins une couche enrobant en totalité une couche sous-jacente et que la réflectance spectrale du pigment résultant s'écarte pour au moins une partie du spectre visible de 200 nm d'étendue, d'au plus 20 % de la réflectance spectrale de référence, mieux d'au plus 10 % et encore mieux d'au plus 2 %.

L'invention a encore pour objet un procédé de fabrication d'une composition à appliquer sur la peau ou les phanères, cette composition comportant, dans un milieu physiologiquement acceptable, au moins un pigment interférentiel ayant une structure multicouche, caractérisé par le fait qu'il comporte les étapes suivantes :
- définir une réflectance spectrale de référence ayant au moins une portion sensiblement confondue avec la réflectance spectrale d'un type de matière kératinique,
- réaliser la structure multicouche de telle sorte qu'elle comporte au moins une couche enrobant en totalité une couche sous-jacente et que la réflectance spectrale de la composition, appliquée sur son support, s'écarte pour au moins une partie du spectre visible de 200 nm d'étendue, d'au plus 20 % de la réflectance spectrale de référence, mieux d'au plus 10 % et encore mieux d'au plus 2 %.

La réflectance spectrale de référence peut être déterminée par exemple à partir d'une information liée à la personne destinée à recevoir la composition, par exemple suite à une réponse à un questionnaire ou à une mesure effectuée sur la peau. La réflectance spectrale de référence peut également être déterminée expérimentalement ou hypothétiquement en utilisant des données composites extraites d'une pluralité de questionnaires, des mesures de la peau, des mesures empiriques, des calculs et autres. La réflectance spectrale de référence peut également être définie arbitrairement. Cependant, elle sera sélectionnée parmi les couleurs de la peau humaine et/ou des cheveux.

L'invention a encore pour objet un procédé de fabrication d'une composition à appliquer sur la peau ou les phanères, cette composition comportant, dans un milieu physiologiquement acceptable, au moins un pigment interférentiel ayant une structure multicouche, caractérisé par le fait qu'il comporte l'étape suivante :
- réaliser la structure multicouche de telle sorte qu'elle comporte au moins une couche enrobant en totalité une couche sous-jacente et que la réflectance spectrale du pigment résultant s'écarte pour au moins une partie du spectre visible de 200 nm d'étendue, d'au plus 20 % de la réflectance spectrale de référence, mieux d'au plus 10 % et encore mieux d'au plus 2 %.

Ce procédé peut également comporter l'étape consistant à prédéfinir la réflectance spectrale de référence. Cette dernière peut être utilisée comme une cible sur laquelle peuvent se fonder les propriétés désirées pour le pigment interférentiel.

L'invention a encore pour objet une composition qui peut comporter :
- un milieu physiologiquement acceptable et, mélangé à ce milieu, au moins un pigment interférentiel ayant une structure multicouche ayant une réflectance spectrale ne s'écartant pas de plus de 20 % d'une réflectance spectrale de référence pour au moins une portion du spectre visible ayant 200 nm d'étendue, ladite structure multicouche comportant au moins une couche enrobant en totalité une couche sous-jacente. La réflectance spectrale de référence peut présenter au moins une portion qui est sensiblement identique à la réflectance spectrale d'un type de matière kératinique.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente isolément et de manière très schématique, un exemple de particule à structure multicouche conforme à un exemple de mise en oeuvre de l'invention,
- la figure 2 représente isolément, de manière très schématique, un autre exemple de particule à structure multicouche selon l'invention,
- la figure 3 représente isolément, de manière très schématique, un exemple de particule à structure multicouche de l'art antérieur, et
- la figure 4 représente la réflectance spectrale de différents types de peau, le pourcentage de lumière réfléchie étant donné en ordonnées et la longueur d'onde en nm en abscisses, ainsi que la réflectance spectrale de différentes compositions cosmétiques réalisées conformément à l'invention.

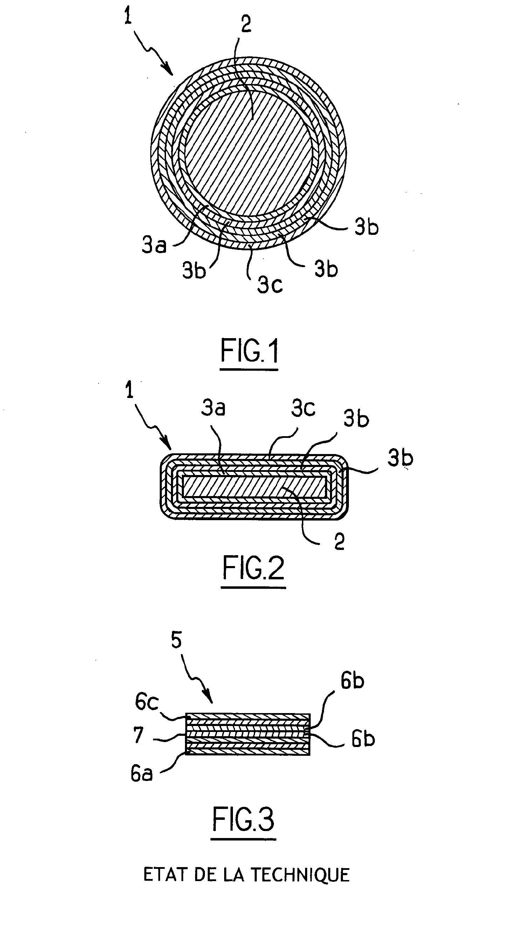

Sur les figures 1 à 3, les proportions relatives réelles des différents éléments n'ont pas été respectées, dans un souci de clarté du dessin.

On a représenté sur la figure 1 une particule 1 de pigment comportant un substrat 2 entouré par une pluralité de couches concentriques, à savoir :
- une couche extrême intérieure 3a déposée sur le substrat 2,
- une couche extrême extérieure 3c, et
- une pluralité de couches intermédiaires 3b entre les couches extrêmes 3a et 3c.

Dans un souci de clarté du dessin, seules trois couches intermédiaires 3b ont été représentées sur la figure 1. Bien entendu, la figure 1 n'est qu'une représentation schématique et le nombre réel de couches intermédiaires peut être différent sans que l'on sorte du cadre de la présente invention, le nombre de couches intermédiaires étant fonction des propriétés optiques que l'on souhaite pour le pigment et pouvant être compris entre 3 et 50, par exemple.

Dans l'exemple considéré, le substrat 2 présente une forme sphérique, étant par exemple constituée par une microsphère pleine en verre.

On ne sort pas du cadre de la présente invention lorsque le substrat présente une forme autre que sphérique, par exemple une forme de plaquette, comme illustré à la figure 2. Par l'expression « substrat en forme de plaquette », on désigne un substrat dont le rapport de la plus grande dimension à l'épaisseur est supérieur ou égal à 5, voire 10 ou 15. L'épaisseur d'un substrat en forme en plaquette est par exemple comprise entre environ 0,5 µm et environ 5 µm.

Le substrat 2 peut être mono-matière ou multi-matériaux, plein ou creux. Le substrat 2 peut être organique ou inorganique. Le substrat peut être naturel mais de préférence on utilise un substrat synthétique, ce qui permet d'avoir plus facilement une forme déterminée.

Le substrat peut être choisi parmi les verres, les céramiques, le graphite, les oxydes métalliques, les alumines, les silices, les silicates, notamment les alumino-silicates et les boro-silicates et le mica synthétique, cette liste n'étant pas limitative.

On a représenté à la figure 3 une particule 5 de pigment selon l'art antérieur.

On voit que celle-ci comporte deux couches extrêmes 6a et 6c et, entre elles, une pluralité de couches intermédiaires 6b, l'empilage des différentes couches 6a, 6b et 6c s'effectuant sans qu'une couche donnée ne recouvre entièrement une couche adjacente, les différentes couches 6a, 6b et 6c ne s'étendant pas sur la tranche 7 de la particule.

En revenant aux figures 1 et 2, les différentes couches 3a, 3b et 3c peuvent être déposées sur le substrat 2 au moyen d'une installation non représentée comportant une chambre d'exposition des particules à un matériau à déposer, chambre dans laquelle les particules sont mises en mouvement par un dispositif vibratoire. Une telle installation est décrite dans l'article « Overcoated Microspheres for Specific Optical Powders » de la revue « Applied Optics », vol. 41, n° 6 du 1^{er} juin 2002.

Bien entendu, le procédé décrit dans cet article n'est qu'un exemple parmi d'autres de procédés qui peuvent être utilisés pour déposer les différentes couches 3a, 3b et 3c d'une particule de pigment.

Les indices de réfraction des différentes couches 3a, 3b et 3c ainsi que leurs épaisseurs respectives sont choisis en appliquant la théorie bien connue des filtres interférentiels, de manière à ce que la réflectance spectrale de la structure multicouche présente les propriétés souhaitées, dans les conditions d'utilisation.

La structure multicouche peut être formée d'une succession de couches de haut et de bas indices de réfraction, les couches de haut indice étant constituées par exemple par un oxyde métallique et les couches de bas indice par un métal.

Il est possible de réaliser avec un nombre relativement faible de couches un pigment interférentiel permettant de réaliser une composition cosmétique ou de soin dont la réflectance spectrale est relativement proche de celle d'un type de peau prédéterminé, caucasien ou autre, afin par exemple de réaliser un maquillage naturel.

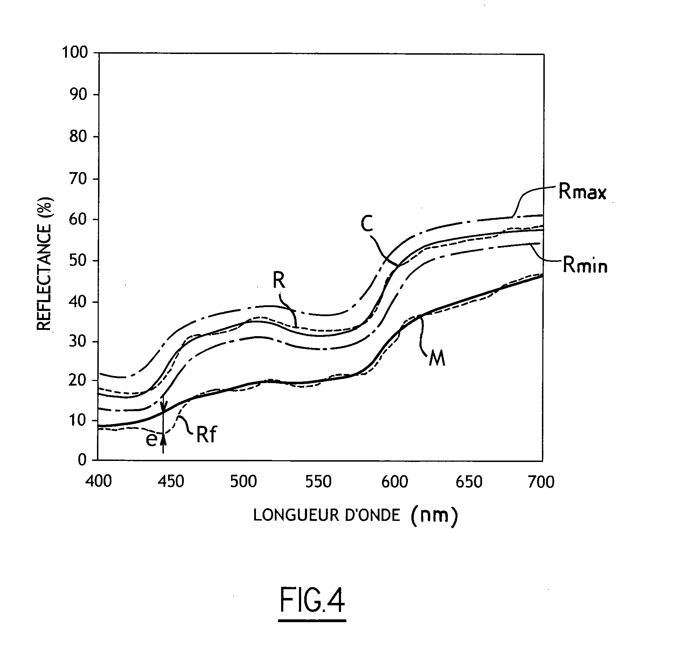

Il faut savoir que d'une manière générale les propriétés optiques de la peau sont différentes selon l'origine du sujet et à titre d'exemple on a représenté à la figure 4 la réflectance spectrale *in vivo* de la peau pour un sujet de type caucasien (courbe C) et métisse (courbe M), en fonction de la longueur d'onde du rayonnement incident. Les mesures sont effectuées à l'aide d'une sphère intégrante disposée sur la peau. On pourra utilement se reporter à l'article « *Spectral Reflectance of human skin in vivo* » P.H. ANDERSEN, P. BJERRING - Photodermatol - Photo imuno - Photomed 1990 : 5-12.

Si l'on cherche à reproduire au mieux la réflectance spectrale d'un type de peau, par exemple caucasien, on réalisera la structure multicouche de telle sorte que la réflectance spectrale R de la composition qui comporte le pigment soit comprise entre des limites minimum Rₘᵢₙ et maximum Rₘₐₓ, ces courbes Rₘᵢₙ et Rₘₐₓ et ne s'écartant par exemple pas de plus de 10 % de la courbe C sur l'ensemble du spectre visible, la courbe C servant dans l'exemple considéré de réflectance spectrale de référence.

Sur la figure 4, on peut ainsi voir que la réflectance R de la composition s'étend dans un « tunnel » délimité par les courbes Rmin et Rmax.

Toutefois, on ne sort pas du cadre de la présente invention lorsque la réflectance spectrale de référence que l'on cherche à reproduire s'écarte sensiblement de la réflectance spectrale d'un type de peau dans une partie donnée du spectre visible, afin d'en corriger certaines dominances chromatiques, par exemple.

La réflectance spectrale de référence peut ainsi être choisie de manière à présenter une réflectance plus faible que celle du type de peau sélectionnée dans le jaune, par exemple.

On a représenté à titre d'illustration sur la figure 4 une réflectance spectrale de référence R_{f} s'écartant sensiblement de la courbe M pour la partie du spectre comprise entre 450 et 500 nm, afin d'atténuer la dominante chromatique jaune d'une peau métisse. L'écart entre la référence spectrale de la composition et la courbe M peut être par exemple supérieur à 5 % environ pour une valeur au moins du spectre.

Un pigment ayant une structure multicouche selon l'invention peut entrer dans la formulation d'un grand nombre de compositions comportant un milieu physiologiquement acceptable, et destinées par exemple au maquillage de la peau, du corps ou du visage.

Le pigment peut représenter entre 0,01 et 50 % en poids par rapport au poids total de la composition, de préférence entre 0,5 et 25 %.

Le milieu physiologiquement acceptable sera adapté à la nature du support sur lequel doit être appliqué la composition ainsi que la forme sous laquelle la composition est destinée à être conditionnée, notamment solide ou fluide à température ambiante et pression atmosphérique.

La composition peut notamment comprendre un milieu cosmétique aqueux et/ou une phase grasse.

La composition peut comprendre de l'eau ou un mélange d'eau et de solvant organique hydrophile, par exemple un alcool.

La composition peut, en outre, comporter des charges. Par « charge », on désigne des particules de toutes formes, insolubles dans le milieu de la composition, quelle que soit la température à laquelle la composition est fabriquée. Ces charges peuvent servir notamment à modifier la rhéologie ou la texture de la composition. La nature et la quantité des corps solides seront fonction des propriétés mécaniques et des textures recherchées. A titre d'exemple de charges, on peut citer entre autres, le talc, le mica, la silice, le kaolin et les poudres de polyamide, par exemple le Nylon® ou l' Orgasol® .

La composition peut également contenir un ou plusieurs actifs cosmétiques, dermatologiques, hygiéniques ou pharmaceutiques.

Comme actifs utilisables dans la composition, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...). Ces actifs peuvent être utilisés par exemple à des concentrations de 0 à 20 % et notamment de 0,001 à 15 %, par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants, les filtres UV, ou leurs mélanges.

La composition cosmétique peut, de plus, comprendre, selon le type d'application envisagée, les constituants classiquement utilisés dans les domaines considérés, qui sont présents en une quantité appropriée à la forme galénique souhaitée.

La composition cosmétique peut se présenter sous toute forme galénique normalement utilisée pour une application topique et notamment sous forme anhydre, sous forme d'une solution huileuse ou aqueuse, d'un gel huileux ou aqueux, d'une émulsion huile-dans-l'eau ou eau-dans-l'huile, d'une émulsion multiple, d'une dispersion d'huile dans de l'eau grâce à des vésicules situées à l'interface huile/eau.

La composition de l'invention peut être sous forme de poudre, de liquide, de solide ou de semi-solide, notamment de produit coulé en stick ou en coupelle, de bâtonnet, de pâte ou de crème plus ou moins fluide.

La composition peut constituer, entre autres, un fond de teint solide ou fluide, un produit anti-cernes ou de contour des yeux, un produit de maquillage du corps ou encore un produit solaire ou de traitement de la peau.

La composition de l'invention peut être obtenue selon les procédés de préparation classiquement utilisés.

L'exemple soumis ci-après est présenté à titre illustratif et non limitatif de l'invention.

On a réalisé un fond de teint destiné à approcher la couleur de peau caucasienne ayant la formulation suivante, en mélangeant de manière classique :

| | |
|---|---|
| Polydiméthyl organosiloxane réticulée dans du polydiméthylsiloxane 6 cs (agent gélifiant et matifiant) | 20 g |
| Cyclopenta-diméthyl siloxane (huile) | 29 g |
| Isoparaffine hydrogénée (huile) | 10g |
| Talc (charge) | 6 g |
| Pigment interférentiel | 10g |
| Hectorite modifiée (argile gélifiant) | q.s.p. 100 g |

Le pigment interférentiel a par exemple la structure suivante :

| COUCHE | MATERIAU | INDICE DE INDICE DE REFRACTION n | EPAISSEUR (nm) |
|---|---|---|---|
| 1 (la plus extérieure) | Al₂O₃ | 1,6726 | 92,1 |
| 2 | Au | 0,7382 | 23,6 |
| 3 | Al₂O₃ | 1,6726 | 81,4 |
| 4 | Au | 0,7382 | 1,8 |
| 5 | Al₂O₃ | 1,6726 | 76,0 |
| 6 | Au | 0,7382 | 13,6 |
| 7 | Al₂O₃ | 1,6726 | 94,6 |
| 8 | Au | 0,7382 | 12,6 |
| SUBSTRAT | Microbille verre 10µm diamètre | 1,5 | |

L'invention permet de réaliser des compositions ayant un fort pouvoir couvrant tout en conférant à la peau maquillée, par exemple, de type caucasien, un aspect naturel, rendant la présence de la composition difficile à détecter.

Il est possible de jouer non seulement sur la réflectance spectrale mais également sur la transmittance spectrale, en choisissant de manière appropriée les différentes couches de la structure multicouche du pigment.

L'invention permet de réaliser des couleurs différentes au moyen d'un ensemble de pigments qui ne diffèrent les uns des autres que par le nombre et les épaisseurs des couches de la structure multicouche. La formulation des compositions cosmétiques et/ou dermatologiques s'en trouve plus facile à établir pour une gamme de teintes qu'avec les pigments de l'art antérieur.

Bien entendu, l'invention n'est pas limitée aux exemples qui viennent d'être décrits. En particulier, on peut réaliser le cas échéant la composition sur-mesure après avoir mesuré la réflectance spectrale de la peau du sujet sur lequel elle est destinée à être appliquée. Dans ce cas, la structure multicouche est calculée de manière à ce que la composition incorporant le pigment s'approche au mieux de la réflectance de la peau du sujet, voire s'en écarte pour certaines valeurs du spectre afin de corriger une dominante chromatique.

Dans toute la description, y compris les revendications, l'expression « comportant un » doit être comprise comme étant synonyme de « comportant au moins un », sauf si le contraire est spécifié.

## Revendications

1. Pigment interférentiel ayant une structure multicouche, **caractérisé par le fait qu'**il présente une réflectance spectrale s'écartant d'au plus 20 % de la réflectance spectrale (C ; M) d'un type de matière kératinique pour au moins une partie du spectre visible de 200 nm d'étendue, ce pigment étant **caractérisé par le fait que** la structure multicouche comporte au moins une couche (3b ; 3c) enrobant en totalité une couche sous-jacente (3a ; 3b).

2. Pigment selon la revendication 1, **caractérisé par le fait que** le pigment comporte des particules (1) présentant une forme générale globulaire, notamment sphérique.

3. Pigment selon la revendication 2, **caractérisé par le fait que** les particules (1) de pigment comportent un substrat (2) ayant une forme globulaire, notamment sphérique, sur lequel sont déposées les différentes couches de la structure multicouche.

4. Pigment selon la revendication précédente, **caractérisé par le fait que** les particules de pigment comportent un substrat (2) composé de microsphères, notamment en verre ou en métal.

5. Pigment selon l'une quelconque des revendications 2 à 4, **caractérisé par le fait que** les particules de pigment présentent une forme symétrique par rapport à un centre de symétrie.

6. Pigment selon l'une quelconque des revendications 2 à 5, **caractérisé par le fait que** la dimension des particules de pigment est comprise entre 10 et 150 µm, mieux entre 10 et 50 µm.

7. Pigment selon l'une quelconque des revendications précédentes, **caractérisé par le fait que** sa réflectance spectrale s'écarte, pour au moins une plage de 200 nm d'étendue, notamment la plage 500-700 nm, d'au plus 10 % de la réflectance spectrale du type de matière kératinique, et mieux d'au plus 2 %.

8. Pigment selon l'une quelconque des revendications précédentes, **caractérisé par le fait qu'**il est configuré de manière à présenter une valeur de clarté L*, mesurée dans l'espace CIE 1976, sensiblement constante pour des incidences comprises entre -45° et 45°.

9. Pigment selon l'une quelconque des revendications précédentes, **caractérisé par le fait que** la matière kératinique est une peau humaine.

10. Composition **caractérisée par le fait qu'**elle comporte, dans un milieu physiologiquement acceptable, au moins un pigment interférentiel ayant une structure multicouche, la réflectance spectrale de la composition s'écartant d'au plus 20 % d'une réflectance spectrale d'un type de matière kératinique pour au moins une partie du spectre visible de 200 nm d'étendue, la structure multicouche comportant au moins une couche (3b ; 3c) enrobant en totalité une couche sous-jacente (3a ; 3c).

11. Composition selon la revendication précédente, **caractérisée par le fait qu'**elle est sensiblement non goniochromatique.

12. Composition selon l'une quelconque des deux revendications immédiatement précédentes, **caractérisée par le fait que** sa réflectance spectrale s'écarte d'au plus 20 % de la réflectance spectrale du type de matière kératinique sur la totalité du spectre visible.

13. Composition selon l'une quelconque des revendications 10 à 12, **caractérisée par le fait que** la composition comporte un pigment tel que défini dans l'une quelconque des revendications 1 à 9.

14. Composition selon l'une quelconque des revendications 10 à 13, **caractérisée par le fait qu'**elle comporte un mélange de pigments, les proportions des différents pigments étant choisies de manière à obtenir la réflectance spectrale souhaitée pour la composition.

15. Composition selon la revendication 14, **caractérisée par le fait qu'**elle comporte un mélange de pigments permettant d'obtenir une réflectance spectrale proche de la réflectance spectrale du type de matière kératinique même en cas de variation de l'indice de réfraction d'une phase de la composition, afin de tenir compte par exemple de l'absorption de sébum par la composition ou de l'évaporation d'un ingrédient.

16. Composition selon l'une quelconque des revendications 10 à 15, **caractérisée par le fait que** la structure multicouche est configurée de manière à ce que la composition soit sensiblement non goniochromatique pour des incidences comprises entre -45° et 45°.

17. Composition selon l'une quelconque des revendications 10 à 16, **caractérisée par le fait que** la réflectance spectrale de la composition appliquée sur son support s'écarte, pour une plage du spectre considérée, notamment pour une plage d'au moins 200 nm d'étendue, notamment pour au moins la plage allant de 500 nm à 700 nm, d'au plus 10 % de la réflectance spectrale de référence et, mieux, d'au plus 5 %, voire encore mieux d'au plus 2 %.

18. Utilisation d'une composition telle que définie dans l'une quelconque des revendications 10 à 17 pour dissimuler des imperfections de la peau, notamment des rides, boutons, taches, couperose, veines et points noirs.

19. Utilisation d'une composition telle que définie dans l'une quelconque des revendications 10 à 17 pour atténuer une dominante chromatique.

20. Utilisation selon la revendication 19, **caractérisée par le fait que** la dominante chromatique qui est atténuée est le jaune.

21. Procédé de fabrication d'un pigment interférentiel ayant une structure multicouche, **caractérisé par le fait qu'**il comporte les étapes suivantes :
- définir une réflectance spectrale de référence ayant au moins une portion sensiblement confondue avec la réflectance spectrale d'un type de matière kératinique,
- réaliser la structure multicouche de telle sorte qu'elle comporte au moins une couche enrobant en totalité une couche sous-jacente et que la réflectance spectrale du pigment s'écarte pour au moins une partie du spectre visible de 200 nm d'étendue, d'au plus 20 % de la réflectance spectrale de référence, mieux d'au plus 10 % et encore mieux d'au plus 2 %.

22. Procédé de fabrication d'une composition à appliquer sur la peau ou les phanères, cette composition comportant, dans un milieu physiologiquement acceptable, au moins un pigment interférentiel ayant une structure multicouche, **caractérisé par le fait qu'**il comporte les étapes suivantes :
- définir une réflectance spectrale de référence ayant au moins une portion sensiblement confondue avec la réflectance spectrale d'un type de matière kératinique,
- réaliser la structure multicouche de telle sorte qu'elle comporte au moins une couche enrobant en totalité une couche sous-jacente et que la réflectance spectrale de la composition, appliquée sur son support, s'écarte pour au moins une partie du spectre visible de 200 nm d'étendue, d'au plus 20 % de la réflectance spectrale de référence, mieux d'au plus 10 % et encore mieux d'au plus 2 %.

23. Procédé de fabrication selon la revendication 22, **caractérisé par le fait que** la réflectance spectrale de référence est déterminée à partir d'une information liée à la personne destinée à recevoir la composition.

24. Procédé de fabrication selon la revendication 23, **caractérisé par le fait que** la réflectance spectrale de référence est déterminée à partir d'une mesure effectuée sur la peau.
